# EUROPÄISCHE PATENTSCHRIFT

(11) **EP 3 273 874 B1**
(45) Veröffentlichungstag und Bekanntmachung des Hinweises auf die Patenterteilung: **28.06.2023**
(21) Anmeldenummer: 16707402.0
(22) Anmeldetag: 19.02.2016
(51) Int. Cl.: A61B 17/12

(54) **VORRICHTUNG ZUM VERSCHLIESSEN EINES HERZOHRS**
DEVICE FOR CLOSING AN ATRIAL APPENDAGE
DISPOSITIF DE FERMETURE D'UN APPENDICE AURICULAIRE CARDIAQUE

(30) Priorität: 27.03.2015 DE 102015104785; 27.03.2015 US 201562139088 P
(43) Veröffentlichungstag der Anmeldung: 31.01.2018
(73) Patentinhaber: PFM Medical AG, 50996 Köln (DE)
(72) Erfinder: JAVOIS, Ales, Hindsdale, Illinois 60521 (US); WACK, Thilo, 66636 Tholey-Hasborn (DE); FREUDENTHAL, Franz, 23000 La Paz (BO)
(74) Vertreter: Hohendorf Kierdorf Patentanwälte PartGmbB
(86) Internationale Anmeldenummer: PCT/EP2016/053548
(87) Internationale Veröffentlichungsnummer: WO 2016/155941

(56) Entgegenhaltungen:
- WO-A1-2007/140797
- WO-A1-2013/032551
- US-A1- 2008 077 180
- US-A1- 2011 054 515
- US-A1- 2015 005 810

## Beschreibung

Die Erfindung betrifft eine Vorrichtung zum Verschließen eines Herzohrs, insbesondere des linken Herzohrs.

Das menschliche Herz ist ein muskuläres Hohlorgan, welches mit rhythmischen Kontraktionen Blut durch den Körper pumpt und so die Versorgung aller Organe sichert. Das menschliche Herz arbeitet wie eine Verdrängerpumpe, in dem Blut ventilgesteuert aus Blutgefäßen angesaugt wird und durch andere Blutgefäße ausgestoßen wird. Das menschliche Herz umfasst zwei kleinere obere Kammern, welche Vorhöfe genannt werden, und zwei größere, kraftvollere Pumpkammern, welche Ventrikel genannt werden. Das Blut fließt durch den rechten Vorhof in das Herz und füllt nachfolgend den rechten Ventrikel. Durch eine Kontraktion des Herzens wird dieses Blut durch die Lungenarterie in die Lunge gepumpt, wo es gefiltert und mit Sauerstoff versorgt wird. Das gefilterte und mit Sauerstoff angereicherte Blut wird in den linken Vorhof gepumpt und füllt anschließend den linken Ventrikel. Von dem linken Ventrikel wird das Blut durch die Aorta in den Körper gepumpt, um Sauerstoff an alle Organe und Zellen zu liefern. Nachdem das Blut den Körperkreislauf durchlaufen hat, ist es wieder sauerstoffarm und kehrt zum Herzen zurück.

Die Herzohren (auriculae atrii) sind Ausstülpungen an den Vorhöfen (atria) des Herzens. Das rechte Herzohr (auricula cordis dextra) liegt neben der aufsteigenden Aorta und das linke Herzohr (auricula cordis sinistra) liegt neben dem Stamm der Lungenarterie (truncus pulmonalis). Das linke Herzohr ist bei Patienten mit Vorhofflimmern ein häufiger Entstehungsort für Blutgerinnsel, welche zu einem Schlaganfall führen können. Das rechte Herzohr ist ein Entstehungsort für eine Thrombenbildung, welche zu einer Lungenembolie führen kann. Die Entstehung von Lungenembolien durch im rechten Vorhof gebildete Gerinnsel ist im Vergleich zu Lungenembolien anderer Ursache jedoch gering.

Das linke Herzohr (left atrial appendage; kurz LAA) ist eine Muskeltasche, die mit dem linken Vorhof des Herzens verbunden ist. Das linke Herzohr ist ein normaler Bestandteil der Anatomie des Herzens und geht auf die embryonale Entwicklung des Herzens zurück. Das linke Herzohr stellt jedoch eine Hauptquelle für die Bildung von Blutgerinnseln bei Patienten mit Vorhofflimmern dar. Derartige Blutgerinnsel können beispielsweise den Blutfluss zum Gehirn blockieren und einen Schlaganfall auslösen, was zu zeitweiligen oder dauerhaften Gehirn- oder Organschäden führen kann.

Beim Vorhofflimmern lösen unregelmäßige elektrische Impulse in den oberen Herzkammern ein vibrieren oder ein zittern aus. Dies hat einen unregelmäßigen und häufig schnellen Herzschlag zur Folge und kann zu verringertem Blutfluss, Herzklopfen und Kurzatmigkeit führen. Darüber hinaus können sich beim Vorhofflimmern Blutgerinnsel im linken Herzohr bilden. Wenn sich der Herzrhythmus normalisiert, können diese Blutgerinnsel vom linken Herzohr in den linken Vorhof und von dort in den sauerstoffreichen Blutstrom gelangen.

Zur Vermeidung von Schlaganfällen bei Vorhofflimmern wird in der modernen Medizin neben einer Medikamentenbehandlung oder einer operativen Entfernung des linken Herzohrs eine Vorrichtung zum Verschluss des Herzohres eingesetzt. Eine Vorbeugung von Schlaganfällen mittels Medikamenten, zum Beispiel mit Vitamin-k-antagonisten, führt zu einer erhöhten Rate an intrazerebralen Blutungen. Die operative Entfernung des linken Herzohrs ist ein schwerer operativer Eingriff und somit mit einem erhöhten Risiko verbunden.

Die zuvor genannten Nachteile werden durch eine Vorrichtung zum Verschließen des linken Herzohres vermieden. Sämtliche Techniken des interventionellen Herzohrverschlusses basieren auf einem venösen, transseptalen Zugang, über welchen eine selbstexpandierende Vorrichtung nach fluoroskopischer Darstellung des linken Herzohres eingebracht wird. Vorteilhaft an einer Verwendung einer Vorrichtung zum Verschließen des linken Herzohres ist, dass diese mittels eines minimalinvasiven Verfahrens implantiert werden kann.

Eine Vorrichtung zum Verschließen eines Herzohres ist grundsätzlich derart auszulegen, dass diese unabhängig von der Anatomie des Herzohres das Herzohr vollkommen verschließt und nach einer erfolgreichen Implantation seine Lage nicht verändert. Dabei kommt es insbesondere auf die Anatomie des Herzohres an. Die Anatomie von Herzohren wird grundsätzlich in drei Typen unterschieden. Beim Typ 1 (Collar) ist der Herzohreingang kleiner als der größte Durchmesser im Herzohr. Bei einem Typ 2 (trichterförmigen) Herzohr ist der Durchmesser des Herzohreingangs größer als der größte Durchmesser im Herzohr und beim Typ 3 (rohrförmigen) Herzohr ist der Herzohreingang in etwa gleich groß wie das dahinter befindliche Herzohr. Alle anderen Typen von Herzohren, wie zum Beispiel blumenkohlartige oder "Chickenwing"-förmige Herzohren, lassen sich unter einen der drei vorgenannten Grundtypen subsummieren.

Die DE 10 2009 036 818 A1 offenbart ein LAA-Okklusionsinstrument bestehend aus einem hochelastischen metallischen Werkstoff mit Gedächtniseigenschaften. Das LAA-Okklusionsinstrument ist mit einer kreisförmigen Abdeckung am proximalen Ende versehen und weist im proximalen Retentionsbereich eine kugelförmige Kupplung mit einem Durchgangsloch auf. Der distale Retentionsbereich des LAA-Okklusionsinstruments besitzt die Form einer Lippe. Hinter dem maximalen Durchmesser des LAA-Okklusionsinstruments ist eine starke Verjüngung an einem sehr beweglichen Steg angeordnet, wobei die Verjüngung so beweglich ist, dass der distale Retentionsbereich des LAA-Okklusionsinstruments bis fast 90° zur kreisförmigen Abdeckung abwinkelbar ist. Zur Fixierung des LAA-Okklusionsinstruments sind am distalen Rand des proximalen Retentionsbereichs Widerhaken vorgesehen. Durch die Verwendung von Widerhaken besteht jedoch grundsätzlich die Gefahr, dass die Gefäßwandung des Herzohrs perforiert wird, wodurch es zu inneren Blutungen kommen kann. Sollte die kreisförmige Abdeckung am proximalen Ende nicht vollumfänglich an der Herzwandung anliegen, verlängert sich der Zeitraum innerhalb welchem das LAA-Okklusionsinstrument mit Endothelgewebe überwuchert wird.

Die DE 10 2010 021 345 A1 betrifft ein Okklusionsinstrument zum Verschließen eines Herzohres, mit einem aus einem Metallrohr gelaserten selbst expandierbaren Occluder. Der Occluder erhält seine Gestalt mittels eines Umformungs- und Wärmebehandlungsverfahrens. Der Occluder weist einen vorderseitigen proximalen Retentionsbereich und einen rückseitigen distalen Retentionsbereich auf, wobei im proximalen Endbereich die gelaserten stabförmigen Elemente in einem Befestigungselement zusammenlaufen. Im zusammengefalteten Zustand lässt sich der Occluder mittels eines Katheters in den Körper eines Patienten minimalinvasiv einführen und in dem Herzohr positionieren. Der Occluder weist einen zwischen dem Mittenbereich zum proximalen Retentionsbereich befindlichen Abschnitt mit Noppen auf, zum Ausbilden einer kraftschlüssigen Verbindung zwischen dem Noppenabschnitt des Occluders und der Herzohrwandung. Die Verwendung von Noppen verringert die Gefahr von Perforationen der Herzohrwandung gegenüber der Verwendung von Widerhaken. Jedoch verwendet das Okklusionsinstrument wie das Okklusionsinstrument gemäß der DE 10 2009 036 818 A1 einen scheibenförmigen proximalen Retentionsbereich, welcher bei einem nicht vollständigen Kontakt mit der Wandung des Herzens deutlich langsamer von Endothelgewebe überwuchert wird.

Aus der DE 10 2012 003 021 A1 ist ein Okklusionsinstrument zum Verschließen eines Herzohrs bekannt, umfassend einen ersten Abschnitt an einem proximalen Ende des Okklusionsinstruments, der an dem Durchmesser eines Herzohreingangs angepasst ist, und ein zweiten Abschnitt, der an die Länge des Herzohrs angepasst ist, wobei der Verschluss des Herzohres durch das Okklusionsinstrument erreicht wird, indem durch zwei in dem zweiten Abschnitt an einem distalen Ende des Okklusionsinstruments übereinander angeordnete Membrane das Okklusionsinstrument in der Länge korrigierbar ist. Zur besseren Verankerung des Okklusionsinstruments sind Widerhakenelemente vorgesehen. Zum Verschluss des Herzohrs weist das Okklusionsinstrument am proximalen Ende einen scheibenförmigen Abschnitt auf. Ferner offenbart die DE 10 2012 003 021 A1 bezüglich eines rohrförmigen Herzohrs, dass das Herzohrinstrument ohne einen scheibenförmigen proximalen Abschnitt auskommt, sofern die Form des Okklusionsinstruments genau der Form des Herzohreingangs entspricht. Nachteilig an dem Okklusionsinstrument ist, dass dieses mittels Hakenelementen in dem Herzohr fixiert wird. Bei der Verwendung eines scheibenförmigen proximalen Abschnitts ergeben sich wiederum die zuvor genannten Probleme bei einer Überwucherung mit Endothelgewebe. Bezüglich der Ausführungsform ohne einen scheibenförmigen proximalen Abschnitt ist nachteilig, dass der proximale Abschnitt in dieser Ausgestaltung genau der Form des Herzohreingangs entsprechen muss.

Die WO 2012/003317 A1 offenbart eine Vorrichtung zum Verschließen eines Herzohrs umfassend eine Occluderscheibe, ausgebildet um im Wesentlichen einen Blutfluss in oder aus dem Herzohr zu vermeiden; einen Mittelbereich mit einem Spiralelement, wobei das Spiralelement mit der Occluderscheibe verbunden ist, ein im Wesentlichen konstanten Querschnitt aufweist und mindestens in der Länge, Orientierung und Winkel zu der Occluderscheibe veränderbar ist; und ein erstes Ankerelement welches mit dem Spiralelement verbunden ist und einen ungleichmäßigen Rand aufweist, welcher derart ausgebildet ist, dass das Okklusionsinstrument an der inneren Wand des Herzohres verankerbar ist ohne das Risiko einer Penetration oder Perforation der Herzohrwandung. Nachteilig an der Okklusionsvorrichtung ist die Verwendung einer Okkluderscheibe, welche bei einer nicht exakten Positionierung zu der Herzwandung erst nach einem längeren Zeitraum von Endothelgewebe überwuchert wird.

Die WO 2007/140797 A1 offenbart ein Okklusionsinstrument zum Verschließen eines Herzohres, mit einem aus einem Geflecht dünner Drähte oder Fäden bestehenden, selbst expandierenden Okklusionskörper, dessen Geflecht mittels eines Umformungs- und Wärmebehandlungsverfahrens eine geeignete Formgebung erhält, wobei der Okklusionskörper einen rückseitigen distalen Retentionsbereich und einen vorderseitigen proximalen Retentionsbereich aufweist, wobei in dem proximalen Retentionsbereich die Enden der Drähte oder Fäden des Geflechts in einer Fassung zusammenlaufen, und wobei der Okklusionskörper ferner einen Mittelbereich zwischen dem distalen und dem proximalen Retentionsbereich aufweist, wobei der Okklusionskörper im zusammengefalteten Zustand mittels eines Katheters in den Körper eines Patienten minimalinvasiv einführbar und in dem Herzohr des Patienten positionierbar ist. Das Okklusionsinstrument umfasst ferner ein Fixiermittel mit einem mittels eines vorzugsweise im Herzohr des Patienten ablaufbaren Polyreaktionsmechanismus gebildeten Polymernetzwerk zum Ausbilden einer kraftschlüssigen Verbindung zwischen dem Geflecht des Okklusionskörpers und der Herzohrwandung. Dabei ist nach der Positionierung des Okklusionskörpers vorgesehen, dass das Fixiermittel insbesondere als niedrig viskose Flüssigkeit, welche durch eine Kanüle applizierbar und kontrolliert zu einem flexiblen unlöslichen Produkt aushärtbar ist, in das Herzohr des Patienten eingefügt wird, damit der Okklusionskörper im Herzohr fest verankert werden kann. Es ist somit notwendig ein niedrig viskoses Fixiermittel nach der Positionierung des Okklusionskörpers in das Herzohr einzufüllen, ohne dass dieses das Herzohr verlässt.

Die WO 2008/125689 A1 offenbart ein Okklusionsinstrument und ein Herstellungsverfahren dazu. Das Okklusionsinstrument besteht aus einem Geflecht aus mindestens einem Draht oder Faden, wobei das Okklusionsinstrument mit einem Umformungs- und/oder Wärmebehandlungsverfahren eine geeignete Formgebung erhalten hat, selbst expandierbar ist, und zur sicheren Verankerung in einem Herzohr des linken oder rechten Vorhofes eines Herzens konfiguriert ist. Das Okklusionselement umfasst einen proximalen Retentionsbereich in der Form einer Scheibe an einem proximalen Ende des Okklusionsinstrumentes; einen distalen Retentionsbereich; und einen Mittelbereich zwischen dem proximalen Retentionsbereich und dem distalen Retentionsbereich; wobei das Okklusionsinstrument ein geschlossenes distales Ende ohne Fassung aufweist und wobei das Okklusionsinstrument wenigstens teilweise im Wesentlichen kugelförmig und hohl ausgeformt ist. Nachteilig an einem derartigen Okklusionsinstrument ist die Verwendung des scheibenförmigen proximalen Retentionsbereiches, welcher bei einer nicht exakten Positionierung erst nach einem längeren Zeitraum mit Endothelgewebe überwuchert wird.

Aus der US 6,290,674 B1 ist eine Vorrichtung zum Verschließen eines Herzohres bekannt. Die Vorrichtung besteht aus einem Gestänge, welches mittels Haken in der Wandung des Herzohres fixiert wird. Die Verwendung von Haken zur Fixierung der Vorrichtung kann jedoch zu inneren Blutungen führen.

Aus der US 2004/0215230 A1 ist eine Okklusionsvorrichtung für ein Herzohr bekannt, welche mittels eines in das Gewebe des Herzohres eingreifendes Element in dem Herzohr fixiert wird. Wie bezüglich des zuvor genannten Okklusionsinstrumentes ausgeführt besteht durch das Eindringen des Elements in das Gewebe des Herzohrs das Risiko von inneren Blutungen.

Die WO 2008/150346 A1 offenbart eine Filtervorrichtung welche das Austreten von Gerinnseln aus einem Herzohr verhindert. Die Vorrichtung wird mittels Haken innerhalb des Herzohres fixiert, was die zuvor genannten Nachteile aufweist.

Die US 2007/027089 A1 offenbart eine Vorrichtung zum Verschließen eines Herzohres. Die Vorrichtung ist spiralförmig ausgebildet und von einer Membran umgeben. Befestigt wird die Vorrichtung innerhalb des Herzohres mittels Halteelementen, welche in das Gewebe des Herzohres eingreifen.

Die aus dem Dokument US 2004/0098031 A1 bekannte Vorrichtung zum Verschließen eines Herzohrs umfasst ebenfalls Fixierungsmittel welche in das Gewebe des Herzohres eingreifen. Ferner verwendet die offenbarte Vorrichtung ein scheibenförmiges Verschlusselement.

Die US 2008/0033241 A1 offenbart eine Vorrichtung zum Verschließen eines Herzohres, welche mittels eines Halteelements in dem Herzohr fixiert wird, wobei das Halteelement in der Gefäßwandung des Herzohrs verankert wird. Ferner wird eine Klemme offenbart, mittels welcher die Herzohröffnung verschlossen wird, wobei die Klemme in die Gefäßwandung des Herzohrs eingreift.

Die US 2005/0113861 A1 offenbart eine Vorrichtung zum Verschließen eines Herzohrs. Die Vorrichtung umfasst ein Stützelement und sich von dem distalen Ende zu dem proximalen Ende der Stütze erstreckende Rippen, wobei die Rippen derart gewölbt sind, dass die Vorrichtung an der Innenwandung des Herzohres anliegt und somit fixiert wird. Über und/oder zwischen den Rippen ist eine Membran gespannt, welche einen Blutfluss in das Herzohr unterbindet. Damit die Vorrichtung sicher in dem Herzohr fixiert wird, muss die Vorrichtung exakt an die Größe und Anatomie des Herzohrs angepasst werden.

Die in der WO 2008/147678 A1 verwendete Vorrichtung wird dazu verwendet ein Herzohr von außen zu greifen und mittels einer umgelegten Schlinge gegenüber dem Vorhof des Herzens abzuschnüren.

Die US 6,652,556 B1 offenbart eine Filtervorrichtung für ein Herzohr. Die Filtervorrichtung wird derart vor der Herzohröffnung platziert, dass Blut im Wesentlichen durch die Filtereinrichtung hindurchtreten kann, während Blutgerinnsel nicht durch die Filtereinrichtung von dem Inneren des Herzohres in die Vorkammer des Herzens gelangen können.

Die WO 2013/032551 A1 offenbart eine weitere Occlusionsvorrichtung für ein Herzohr, welche mittels Widerhaken an Verankerungselementen in dem Herzohr fixiert wird.

Der vorliegenden Erfindung liegt die Aufgabe zugrunde die aus dem Stand der Technik bekannten Nachteile zu vermeiden und insbesondere eine Vorrichtung zum Verschließen eines Herzohres bereitzustellen welche unabhängig von der Anatomie des Herzohrs sicher im Herzohr verankert werden kann, ohne das Gewebe des Herzohrs zu beschädigen, und schnellstmöglich von Endothelgewebe überwuchert wird.

Die Aufgabe wird erfindungsgemäß gelöst durch eine Vorrichtung zum Verschließen eines Herzohrs, insbesondere des linken Herzohrs, gemäß Anspruch 1.

Eine erfindungsgemäße Vorrichtung umfasst ein Verschlusselement, ausgebildet um in einem Herzohreingang angeordnet zu werden und einen Eintritt von Blut in das Herzohr und/oder einen Austritt von Thromben aus dem Herzohr heraus zu verhindern, welches derart ausgebildet ist, dass es im implantierten Zustand nicht aus dem Herzohr in den Vorhof des Herzens herausragt; ein erstes Verankerungselement, welches benachbart zu dem Verschlusselement angeordnet und mit dem Verschlusselement verbunden ist, wobei das erste Verankerungselement zur Verankerung an einer Herzohrwandung ausgebildet ist; ein zumindest in radialer Richtung flexibel ausgebildetes Verbindungselement, welches mit dem ersten Verankerungselement verbunden ist; und ein zweites Verankerungselement, welches mit dem Verbindungselement verbunden ist und zur Verankerung an der Herzohrwandung ausgebildet ist.

Die Verwendung eines in dem Herzohreingang angeordneten Verschlusselements gegenüber der Verwendung eines scheibenförmigen Retentionsbereiches, welcher in dem Vorhof des Herzens vor dem Herzohreingang angeordnet ist hat den Vorteil, dass das Verschlusselement mit seinem gesamten Umfang an der Wandung des Herzohreingangs anliegt. Dadurch wird gewährleistet, dass das Verschlusselement schnellstmöglich mit Endothelgewebe überwuchert werden kann. Im Gegensatz dazu ist bei der Verwendung eines scheibenförmigen Retentionsbereiches nicht gewährleistet, dass der scheibenförmige Retentionsbereich mit seinem vollen Umfang an der Herzwandung im Bereich der Herzohröffnung anliegt. In den Bereichen in welchen der scheibenförmige Retentionsbereich nicht an der Herzwandung anliegt findet eine Überwucherung mit Endothelgewebe erst statt, wenn derartiges Endothelgewebe von anderen Bereichen in dem Bereich des scheibenförmigen Retentionsbereichs hineinwächst im welchen kein Kontakt zu der Herzwandung besteht. Ferner wird durch das Verschlusselement sichergestellt, dass keine Thromben aus dem Herzohr zwischen Verschlusselement und Herzwandung in den Vorhof des Herzens wandern.

Das erste Verankerungselement, welches benachbart zu dem Verschlusselement angeordnet und mit dem Verschlusselement verbunden ist, stellt sicher, dass das Verschlusselement sicher im Herzohreingang gehalten wird und das Verschlusselement nicht aus dem Herzohreingang in den Vorhof des Herzens heraustritt.

Das zweite Verankerungselement dient zur Verbesserung der Verankerung der Vorrichtung in dem Herzohr. Das zweite Verankerungselement ist über ein Verbindungselement mit dem ersten Verankerungselement verbunden. Das Verbindungselement ist in radialer Richtung flexibel ausgebildet, da sich die Anatomie von Herzohren untereinander unterscheidet und insbesondere ein Herzohr üblicherweise nicht senkrecht von der Herzwandung absteht sondern an dieser anliegt, dabei jedoch zumeist in sich abgewinkelt ist.

Das erste Verankerungselement und das zweite Verankerungselement sind zur Verankerung an der Herzwandung ausgebildet. Eine Verankerung an der Herzohrwandung im Sinne der Erfindung schließt eine Verankerung in der Herzohrwandung, also eine Perforation der Herzohrwandung, aus.

Nach der Erfindung ist der Durchmesser des Verschlusselements größer als der Herzohreingang. Der Durchmesser des Verschlusselements ist zwischen 5% und 20% größer als der Herzohreingang, vorzugsweise ungefähr 10%. Dadurch, dass der Durchmesser des Verschlusselements größer ist als der Herzohreingang, ist gewährleistet, dass sich das Verschlusselement vollumfänglich an die Wandung des Herzohreingangs anlegt. Ferner wird dadurch eine zusätzliche Fixierung und Zentrierung des Verschlusselements in dem Herzohreingang erzielt.

Gemäß einer Variante der Erfindung umfasst das Verschlusselement ein erstes Membranelement, wobei das erste Membranelement undurchlässig für Thromben ist. Beispielsweise ist das erste Membranelement porös ausgebildet, welches einen Blutfluss zwischen Herzohr und Vorhof nicht verhindert, aber einen Übergang von Thromben von dem Herzohr in den Vorhof des Herzens verhindert. Ferner fördert ein porös ausgebildetes erstes Membranelement aufgrund der geringen Porengröße ein Überwuchern des ersten Membranelements mit Gewebe. Alternativ kann das erste Membranelement auch undurchlässig für Flüssigkeiten ausgebildet sein.

Nach einer alternativen oder zusätzlichen Variante der Erfindung umfasst das Verschlusselement ein zweites Membranelement, wobei das zweite Membranelement ausgebildet ist um ein Überwuchern des Verschlusselements mit Gewebe zu fördern. Durch ein Überwuchern des Verschlusselements mit Gewebe wird das Verschlusselement einerseits sicher in dem Herzohreingang fixiert und andererseits wird die Dichtigkeit des Verschlusselements hinsichtlich eines Blutflusses aus dem Herzohr heraus über einen unbestimmten Zeitraum sichergestellt.

Zweckmäßigerweise sind das erste Membranelement und das zweite Membranelement einstückig ausgebildet. Durch die einstückige Ausbildung des ersten Membranelements und des zweiten Membranelements muss lediglich ein Element mit dem Verschlusselement verbunden werden, beispielsweise durch kleben, schweißen, nähen, aufschmelzen, tauchen oder einem anderen Fügeverfahren. Vorzugsweise ist das erste Membranelement und/oder das zweite Membranelement oder das einstückige Membranelement unlösbar mit dem Verschlusselement verbunden.

Zweckmäßigerweise sind das Verschlusselement und das erste Membranelement und/oder das zweite Membranelement oder das einstückige Membranelement lösbar oder unlösbar miteinander verbunden oder verbindbar, insbesondere durch Kleben, Schweißen, Nähen, Aufschmelzen, Tauchbeschichten oder einem anderen Fügeverfahren.

Ferner kann das erste Membranelement und/oder das zweite Membranelement oder das einstückige Membranelement nach einer Variante der Erfindung mit einer Beschichtung versehen sein, zum Schaffen einer Biostabilität und/oder Biokompatibilität. Biostabilität im Sinne der Erfindung bezieht sich auf die biologischen, chemischen und/oder physikalischen Belastungen am Implantationsort. Biokompatibilität im Sinne der Erfindung betrifft die Integration der Vorrichtung in die biologische Umgebung am Implantationsort, vorzugsweise ohne durch die Materialbeschaffenheit, Form und Funktion das umliegende Gewebe zu irritieren.

Beispielsweise umfasst das erste Membranelement und/oder das zweite Membranelement oder das einstückige Membranelement mindestens eins der folgenden Materialien: Polyester, Polytetrafluorethylen (PTFE), extrudiertes/expandiertes PTFE (ePTFE), Teflon, Filz, Goretex, Silikonurethan, Metallfasern und Polypropylen. Im Allgemeinen kann das erste Membranelement und/oder das zweite Membranelement oder das einstückige Membranelement aus einem synthetischen und biologischen Material bestehen, insbesondere aus einem bioresorbierbaren Material.

Nach einer besonders zweckmäßigen Variante der Erfindung decken das erste Membranelement und/oder das zweite Membranelement oder das einstückige Membranelement im implantierten Zustand der Vorrichtung den Herzohreingang vollständig ab. Somit wird die Dichtigkeit des Herzohreingangs hinsichtlich eines Übertritts von Thromben aus dem Herzohr heraus beziehungsweise ein überwuchern des gesamten Herzohreingangs mit Gewebe gewährleistet.

Nach einer weiteren Variante der Erfindung umschließt das erste Membranelement und/oder das zweite Membranelement oder das einstückige Membranelement den Rand des Verschlusselements derart, dass das erste Membranelement und/oder das zweite Membranelement oder das einstückige Membranelement im implantierten Zustand der Vorrichtung vollständig an der Herzohrwandung anliegt. Die direkte Verbindung zwischen Membranelement und Herzohrwandung verbessert zum einen die Dichtigkeit des Herzohreingangs und fördert weiterhin das Überwuchern des Membranelements beziehungsweise des Verschlusselements mit Gewebe. Beispielsweise umschließt das erste Membranelement und/oder das zweite Membranelement oder das einstückige Membranelement den Rand des Verschlusselements und erstreckt sich in Richtung des ersten Verankerungselements, zum Beispiel bis zur Mitte des Verschlusselements.

Erfindungsgemäß ist das Verschlusselement zylindrisch, kegelförmig, konisch zulaufend, kegelstumpfförmig, zylindrisch-kegelförmig, zylindrisch-konisch zlaufend oder zylindrisch-kegelstumpfförmig ausgebildet. Das Verschlusselement erstreckt sich über eine Länge von 0,5 cm bis 2,5 cm in Längsrichtung der Vorrichtung. Dies hat den Vorteil, dass der Kontaktbereich zwischen Verschlusselement und Herzohreingang vergrößert wird, wodurch die Fixierung des Verschlusselements verbessert wird und ferner die Dichtigkeit gewährleistet wird und die Überwucherung des Verschlusselements mit Gewebe gefördert wird.

Gemäß der Erfindung ist die dem Vorhof des Herzens zugewandte Oberfläche des Verschlusselements eben ausgebildet. Es ragen somit keine Bereiche der erfindungsgemäßen Vorrichtung in den Vorhof des Herzens hinein.

Nach einer bevorzugten Variante der Erfindung sind das Verschlusselement und das erste Verankerungselement fest miteinander verbunden oder einstückig miteinander ausgebildet.

Das Verbindungselement ist vorzugsweise mittels einer ersten Verbindung mit dem ersten Verankerungselement verbunden und/oder mittels einer zweiten Verbindung mit dem zweiten Verankerungselement verbunden. Zweckmäßigerweise sind die erste Verbindung und die zweite Verbindung an gegenüberliegenden Enden des Verbindungselements angeordnet. Generell ist das erste Verankerungselement an einem Ende des Verbindungselements angeordnet und das zweite Verbindungselement an einem gegenüberliegenden Ende des Verbindungselements angeordnet. Die erste Verbindung und/oder die zweite Verbindung sind vorzugsweise derart ausgebildet, dass sie sich nach der Implantation nicht mehr lösen können.

Nach einer zweckmäßigen Variante der Erfindung ist das Verbindungselement zylindrisch ausgebildet. Durch die zylindrische Ausbildung des Verbindungselements kann insbesondere die Flexibilität in radialer Richtung gewährleistet werden. Ferner kann ein derartiges zylindrisches Verbindungselement auf einfache Art und Weise durch Schneiden, insbesondere Laserschneiden, aus einem Rohr hergestellt werden.

Beispielsweise ist das Verbindungselement wendelförmig ausgebildet.

Gemäß der Erfindung ist das Verbindungselement aus einem Rohr hergestellt, insbesondere durch Schneiden von Durchbrechungen in dem Rohr, beispielsweise mittels eines Lasers. Beispielsweise lässt sich somit aus dem Rohr ein spiralförmiges Verbindungselement Schneiden, welches zylindrisch ausgebildet ist und in radialer Richtung flexibel ist.

In einer besonders bevorzugten Variante der Erfindung ist das Verschlusselement, das erste Verankerungselement und/oder das zweite Verankerungselement aus einem einzigen drahtartigen Element oder mehreren drahtartigen Elementen durch verschränkendes Wickeln und/oder Verbinden und/oder Verflechten nach Art eines Gewebes und/oder Geleges und/oder Netzes geformt. Dies gewährleistet im implantierten Zustand eine hohe Stabilität, wobei die Vorrichtung während der Implantation gleichzeitig flexibel ist, so dass die Vorrichtung beispielsweise mittels eines minimalinvasiven Verfahrens implantierbar ist. Beispielsweise sind das Verschlusselemente und das erste Verankerungselement aus einem einzigen drahtartigen Element oder mehreren drahtartigen Elementen hergestellt und das zweite Verankerungselement ist aus einem weiteren einzigen drahtartigen Element oder mehreren drahtartigen Elementen hergestellt. Alternativ können das Verschlusselement, das erste Verankerungselement und das zweite Verankerungselement aus einem einzigen drahtartigen Element oder mehreren drahtartigen Elementen hergestellt sein. Ein einziges drahtartiges Element kann dabei auch aus mehreren Elementen beispielsweise in Form einer Litze oder parallel zueinander verlaufenden Einzeldrähten gebildet sein. Bei der Verwendung von mehreren drahtartigen Elementen sind diese vorzugsweise an einer Stelle der Vorrichtung in einer Halterung zusammengefasst. Die drahtartigen Elemente weisen beispielsweise einen runden oder ovalen Querschnitt auf.

Ferner kann die Vorrichtung mehrere Lagen eines Gewebes und/oder Geleges und/oder Netzes aufweisen.

Nach einer weiteren bevorzugten Variante der Erfindung bestehen das Verschlusselement, das erste Verankerungselement und das zweite Verankerungselement aus einem Formgedächtnismaterial, insbesondere aus Nitinol oder einem Kunststoff mit Formgedächtnis. Die Verwendung des Formgedächtnismaterials erleichtert insbesondere die minimalinvasive Implantation der erfindungsgemäßen Vorrichtung.

Gemäß einer weiteren erfindungsgemäßen Variante umfasst die Vorrichtung eine röntgendichte Markierung, insbesondere in Form von Markerplaketten, Markierungen oder Markerdrähten. Somit lässt sich während der Implantation der erfindungsgemäßen Vorrichtung die Positionierung der Vorrichtung mit einem bildgebenden Verfahren wie zum Beispiel Röntgen oder MRT überprüfen.

In einer weiteren Variante der Erfindung ist das erste Verankerungselement und/oder das zweite Verankerungselement scheibenförmig oder schirmförmig ausgebildet und erstreckt sich vorzugsweise in radialer Richtung der Vorrichtung. Insbesondere ist das erste Verankerungselement und/oder das zweite Verankerungselement bauchig oder gekrümmt ausgebildet. Derartige Ausgestaltungen eignen sich besonders zur Verankerung der Vorrichtung an der Herzohrwandung.

Gemäß einer Variante der Erfindung ist der Durchmesser des ersten Verankerungselements gleich dem Durchmesser des zweiten Verankerungselements. Vorzugsweise ist der Durchmesser des ersten Verankerungselements größer als der Durchmesser des zweiten Verankerungselements, da sich der Durchmesser des Herzohrs in Richtung gegenüberliegend zu der Herzohröffnung verjüngt.

Nach einer besonders bevorzugten Variante der Erfindung weist das erste Verankerungselement und/oder das zweite Verankerungselement einen ersten Teilabschnitt und einen zweiten Teilabschnitt auf, wobei sich der erste Teilabschnitt in radialer Richtung nach außen erstreckt und der zweite Teilabschnitt in radialer Richtung nach innen zurückgefaltet ist, insbesondere derart, dass der erste Teilabschnitt und der zweite Teilabschnitt doppellagig aufeinander gefaltet sind. Ein doppellagiges Verankerungselement weist eine erhöhte Stabilität auf und somit eine verbesserte Verankerung an der Herzohrwandung. Insbesondere entfaltet sich der zweite Teilabschnitt während der Implantation zuerst und faltet sich während sich der erste Teilabschnitt radial nach außen erstreckt zurück auf den ersten Teilabschnitt. Die sequenzielle Entfaltung des ersten Teilabschnitts und des zweiten Teilabschnitts hat den Vorteil, dass der für die Implantation benötigte Freiraum minimiert wird. Ferner hat die erste Entfaltung des zweiten Teilabschnitts und nachfolgende Rückfaltung auf den ersten Teilabschnitt den Vorteil, dass der zweite Teilabschnitt in die Muskelstränge innerhalb des Herzohrs eingreifen kann, wodurch die Vorrichtung besser in dem Herzohr fixiert wird.

Nach einer weiteren erfindungsgemäßen Variante ist das erste Verankerungselement und/oder das zweite Verankerungselement aufgewickelt, vorzugsweise schneckenförmig. Dies erhöht ebenfalls die Stabilität des Verankerungselements und somit wird die Verankerung an der Herzohrwandung weiter verbessert.

In einer zweckmäßigen Variante der Erfindung ist die Zurückfaltung oder Aufwicklung des ersten Verankerungselements und/oder des zweiten Verankerungselements in Richtung des Verschlusselements angeordnet. Somit wird insbesondere ein Verschieben der erfindungsgemäßen Vorrichtung in Richtung der Herzohröffnung vermieden.

Nach einer weiteren bevorzugten Variante der Erfindung wird der äußere Rand des ersten Verankerungselements und/oder des zweiten Verankerungselements durch Schlingen oder Schlaufen gebildet. Die Schlingen oder Schlaufen sind dabei nebeneinander oder teilweise übereinander überlappend ausgebildet, so dass sich ein wellenförmiger äußere Rand des ersten Verankerungselements und/oder des zweiten Verankerungselements ergibt. Der wellenförmige äußere Rand des ersten Verankerungselements und/oder des zweiten Verankerungselements bewirkt eine bessere Verankerung der erfindungsgemäßen Vorrichtung an der Herzohrwandung.

Zweckmäßigerweise wird der äußere Rand des ersten Verankerungselements und/oder des zweiten Verankerungselements durch Schlingen oder Schlaufen gleicher Größe gebildet. Daraus ergibt sich ein gleichförmiger wellenförmiger äußere Rand des ersten Verankerungselements und/oder des zweiten Verankerungselements.

Alternativ wird der äußere Rand des ersten Verankerungselements und/oder des zweiten Verankerungselements durch Schlingen oder Schlaufen unterschiedlicher Größe gebildet. Vorzugsweise ist die Anordnung der unterschiedlichen Größen der Schlingen oder Schlaufen gleichmäßig, beispielsweise eine große Schlinge oder Schlaufe nach zwei oder drei kleinen Schlingen oder Schlaufen. Insbesondere die Kombination mit einer Zurückfaltung eines Teilabschnitts des ersten Verankerungselements und/oder des zweiten Verankerungselements zu dem anderen Teilabschnitt des ersten Verankerungselements und/oder zweiten Verankerungselements wird eine verbesserte Fixierung der erfindungsgemäßen Vorrichtung an der Herzohrwandung erzielt, da die größeren Schlingen oder Schlaufen bei der Zurückfaltung in die Muskelstränge innerhalb des Herzohres eingreifen können.

Gemäß einer zweckmäßigen Variante der Erfindung ist die Vorrichtung replatzierbar und/oder explantierbar ausgebildet, beispielsweise durch ein Kopplungselement für ein Implantationsinstrument.

In einer weiteren erfindungsgemäßen Variante weist die Vorrichtung in einem ersten Betriebszustand (Primärform) ein großes Verhältnis von Länge zu Querausdehnung entlang der Längsachse der Vorrichtung und in einem zweiten Betriebszustand (Sekundärform) ein kleineres Verhältnis von Länge zu Querausdehnung entlang der Längsachse der Vorrichtung auf und die Vorrichtung ist durch Aufbringen einer Kraft gegen elastische Materialkräfte aus der Sekundärform in die Primärform reversibel überführbar. Die erfindungsgemäße Vorrichtung lässt sich dadurch einfach mittels eines minimalinvasiven Verfahrens innerhalb des Herzohrs eines Patienten implantieren.

Nachfolgend wird die Erfindung anhand von in den Figuren dargestellten Ausführungsbeispielen näher erläutert. Es zeigen:
- Fig. 1:: eine perspektivische Ansicht einer erfindungsgemäßen Vorrichtung,
- Fig. 2:: eine Detailansicht eines Verschlusselements,
- Fig. 3:: eine Detailansicht eines Verbindungselements,
- Fig. 4:: eine Detailansicht eines zweiten Verankerungselements,
- Fig. 5:: eine schematische Ansicht eines weiteren Verschlusselements,
- Fig. 6:: eine schematische Ansicht eines weiteren zweiten Verankerungselements,
- Fig. 7:: eine schematische Ansicht einer Struktur eines Verschlusselements und/oder Verankerungselements,
- Fig. 8:: eine schematische Ansicht einer weiteren Struktur eines Verschlusselements und/oder Verankerungselements,
- Fig. 9:: eine schematische Ansicht von Ausgestaltungen eines drahtartigen Elements,
- Fig. 10:: eine schematische Ansicht eines doppellagigen Verschlusselements und/oder Verankerungselements,
- Fig. 11:: eine schematische Ansicht eines mehrlagigen Verschlusselements und/oder Verankerungselements,
- Fig. 12:: eine schematische Ansicht einer ersten Ausgestaltung eines äußeren Randes eines Verankerungselements,
- Fig. 13:: eine schematische Ansicht einer zweiten Ausgestaltung eines äußeren Randes eines Verankerungselements, und
- Fig. 14:: eine schematische Ansicht einer dritten Ausgestaltung eines äußeren Randes eines Verankerungselements.

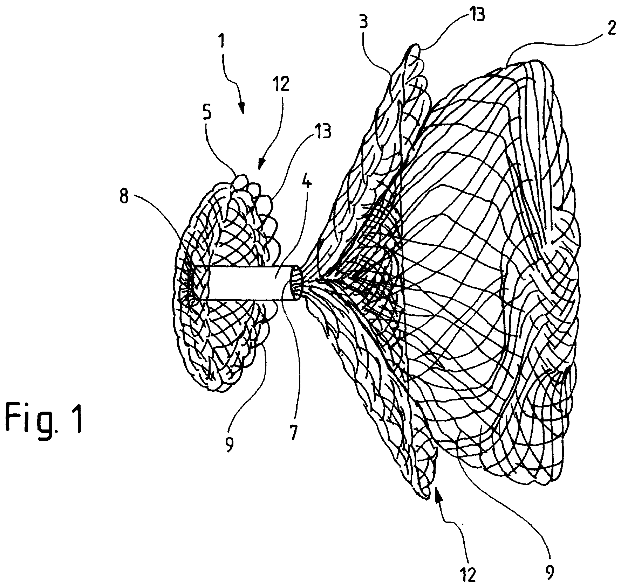

Fig. 1 zeigt eine perspektivische Ansicht einer erfindungsgemäßen Vorrichtung 1 zum Verschließen eines Herzrohrs, insbesondere des linken Herzohrs. Die Vorrichtung 1 umfasst ein Verschlusselement 2, ausgebildet um in einem Herzohreingang angeordnet zu werden und einen Eintritt von Blut in das Herzohr und/oder einen Übertritt von Thromben aus dem Herzohr heraus zu verhindern. Das Verschlusselement 2 ist derart ausgebildet, dass es im implantierten Zustand nicht aus dem Herzohr in den Vorhof des Herzens herausragt.

Die Vorrichtung 1 umfasst ferner ein erstes Verankerungselement 3, welches benachbart zudem Verschlusselement 2 angeordnet und mit dem Verschlusselement 2 verbunden ist, wobei das erste Verankerungselement 3 zur Verankerung an einer Herzohrwandung ausgebildet ist.

Die Vorrichtung 1 umfasst weiterhin ein zumindest in radialer Richtung flexibel ausgebildetes Verbindungselement 4, welches mit dem ersten Verankerungselement 3 verbunden ist.

Ferner umfasst die Vorrichtung 1 ein zweites Verankerungselement 5, welches mit dem Verbindungselement 4 verbunden ist und zur Verankerung an der Herzohrwandung ausgebildet ist.

Der Durchmesser des Verschlusselements 2 ist zwischen 5% und 20% größer als der Herzohreingang, vorzugsweise ungefähr 10%.

Das Verschlusselement 2 ist im Bereich des Herzohreingangs kegelstumpfförmig ausgebildet und weist im weiteren Verlauf in Richtung des hinteren Herzohrs einen konisch zulaufenden Bereich auf. Die dem Vorhof des Herzens zugewandte Oberfläche des Verschlusselements 2 ist eben ausgebildet.

Nachfolgend zu dem konisch zulaufenden Bereich des Verschlusselements 2 ist das erste Verankerungselement 3 angeordnet. In dem Ausführungsbeispiel gemäß Fig. 1 sind das Verschlusselement 2 und das erste Verankerungselement 3 einstückig ausgebildet.

Das Verbindungselement 4 ist mittels einer ersten Verbindung 7 mit dem ersten Verankerungselement verbunden und mittels einer zweiten Verbindung 8 mit dem zweiten Verankerungselement 5 verbunden. Die erste Verbindung 7 und die zweite Verbindung 8 sind an gegenüberliegenden Enden des Verbindungselements 4 angeordnet.

Wie der Fig. 1 entnommen werden kann, ist das Verbindungselement 4 zylindrisch und spiralförmig ausgebildet.

Das Verschlusselement 2 und das erste Verankerungselement 3 sind einstückig ausgebildet und aus einem einzigen drahtartigen Element 9 durch verschränkendes Wickeln und/oder Verwinden und/oder Verflechten nach Art eines Gewebes und/oder Geleges und/oder Netzes geformt. Das zweite Verankerungselement 5 ist ebenfalls aus einem einzigen drahtartigen Element 9 durch verschränkendes Wickeln und/oder Verwinden und/oder Verflechten nach Art eines Gewebes und/oder Geleges und/oder Netzes geformt. Die drahtartigen Elemente 9 zur Ausbildung des Verschlusselements 2 und der ersten Verankerungselements 3 beziehungsweise des zweiten Verankerungselements 5 bestehen aus einem Formgedächtnismaterial. In dem Ausführungsbeispiel gemäß Fig. 1 bestehen die drahtartigen Elemente 9 aus Nitinol.

Die Vorrichtung 1 aus Fig. 1 kann weiterhin eine röntgendichte Markierung umfassen, insbesondere in Form von Markerplaketten, Markierung oder Markerdrähten. Mittels der röntgendichten Markierung kann die Positionierung der Vorrichtung 1 während der Implantation mit einem bildgebenden Verfahren wie zum Beispiel Röntgen oder Magnetresonanztomographie (MRT) überprüft werden.

Wie ebenfalls der Fig. 1 entnommen werden kann erstrecken sich das erste Verankerungselement 3 und das zweite Verankerungselement 5 in radialer Richtung. Das erste Verankerungselement 3 und das zweite Verankerungselement 5 sind dabei schirmförmig ausgebildet, wobei der Hohlraum des Schirms in Richtung des Herzohreingangs bzw. des Verschlusselements 2 angeordnet ist. Alternativ könnten das erste Verankerungselement 3 und/oder das zweite Verankerungselement 5 auch bauchig und/oder gekrümmt ausgebildet werden. Wobei der durch die bauchige und/oder gekrümmte Ausbildung entstehende Hohlraum ebenfalls in Richtung des Herzohreingangs bzw. des Verschlusselements 2 angeordnet ist.

Der Durchmesser des ersten Verankerungselements 3 ist größer als der Durchmesser des zweiten Verankerungselements 5, da sich ein Herzohr üblicherweise mit größerer Entfernung von dem Herzohreingang verjüngt und das erste Verankerungselement 3 näher an dem Herzohreingang angeordnet ist als das zweite Verankerungselement 5.

Der äußere Rand 12 des ersten Verankerungselements 3 und des zweiten Verankerungselements 5 wird durch Schlingen oder Schlaufen 13 gebildet. In dem Ausführungsbeispiels gemäß Fig. 1 wird der äußere Rand 12 des ersten Verankerungselements 3 und des zweiten Verankerungselements 5 durch Schlingen oder Schlaufen 13 gleicher Größe gebildet, woraus sich ein wellenförmiger äußerer Rand 12 ergibt.

Die Vorrichtung 1 zum Verschließen eines Herzohres, insbesondere des linken Herzohres, aus Fig. 1 weist in einem ersten Betriebszustand (Primärform) ein großes Verhältnis von Länge zu Querausdehnung entlang der Längsachse der Vorrichtung 1 und in einem zweiten Betriebszustand (Sekundärform) ein kleineres Verhältnis von Länge zu Querausdehnung entlang der Längsachse der Vorrichtung 1 auf. Die Vorrichtung 1 ist durch Aufbringen einer Kraft gegen elastische Materialkräfte aus der Sekundärform in die Primärform reversibel überführbar. Fig. 1 zeigt die Vorrichtung 1 in der Sekundärform. Durch Aufbringen einer Kraft gegen elastische Materialkräfte ist die Vorrichtung 1 aus Fig. 1 reversibel in die Primärform überführbar. In der Primärform kann die erfindungsgemäße Vorrichtung 1 über einen Katheter minimalinvasiv in einem Herzohr eines Patienten implantiert werden. Dabei wird die Vorrichtung 1 in Richtung der Längsachse der Vorrichtung 1 gedehnt, wodurch die Vorrichtung 1 eine im Wesentlichen zylindrische Form annimmt. In dieser zylindrischen Form wird die Vorrichtung 1 über einen Katheter bis in das Herzohr bewegt. Sobald die Vorrichtung 1 aus dem distalen Ende des Katheters austritt nimmt die Vorrichtung 1 wieder die Sekundärform an. Dabei wird zunächst das zweite Verankerungselement 5 aus dem distalen Ende des Katheters herausgeführt, wobei sich das zweite Verankerungselement 5 an der Herzohrwandung fixiert. Im weiteren Verlauf der Implantation der erfindungsgemäßen Vorrichtung 1 tritt das Verbindungselement 4 aus dem distalen Ende des Katheters aus. Nachfolgend tritt das erste Verankerungselement 3 aus dem distalen Ende des Katheters aus und verankert sich an der Herzohrwandung. Abschließend tritt das Verschlusselement 3 aus dem distalen Ende des Katheters aus und wird in dem Herzohreingang platziert um einen Eintritt von Blut in das Herzohr und/oder einen Übertritt von Thromben aus dem Herzohr heraus zu verhindern. In dem implantierten Zustand ragt das Verschlusselement 2 nicht aus dem Herzohr in den Vorwurf des Herzens heraus. Die genaue Positionierung der erfindungsgemäßen Vorrichtung 1 kann mittels eines bildgebenden Verfahrens wie Röntgen oder Magnetresonanztomographie überprüft werden. In Abhängigkeit von dem Material der erfindungsgemäßen Vorrichtung 1 kann die erfindungsgemäße Vorrichtung 1 gegebenenfalls eine oder mehrere röntgendichte Markierungen umfassen.

Nachdem die Positionierung der erfindungsgemäßen Vorrichtung 1 mit bildgebenden Verfahren überprüft wurde, wird die Verbindung zwischen der erfindungsgemäßen Vorrichtung 1 und den zur Implantation verwendeten Instrumenten gelöst, so dass die erfindungsgemäße Vorrichtung 1 in dem Herzohr verbleibt und in den folgenden Wochen/Monaten schnellstmöglich von den umliegenden Gewebe überwuchert wird, insbesondere im Bereich des Herzohreingangs.

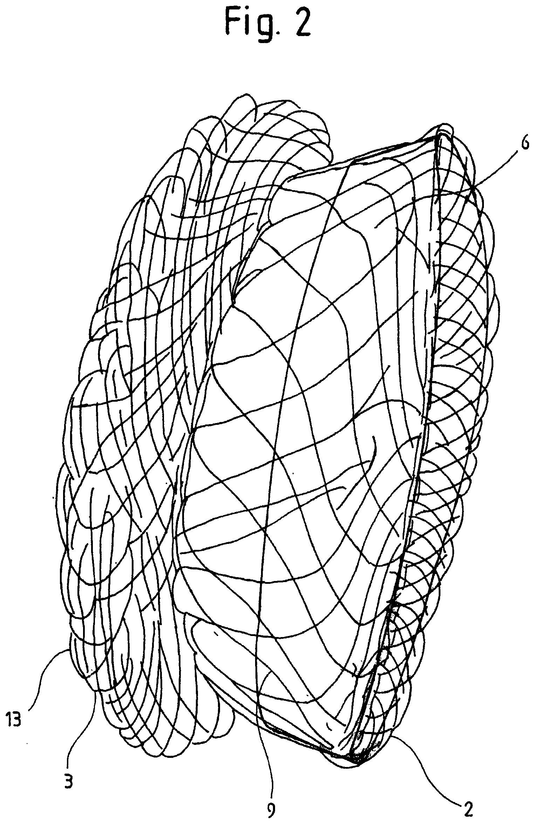

Fig. 2 zeigt eine Detailansicht eines Verschlusselements 2 zur Verwendung mit einer erfindungsgemäßen Vorrichtung 1. Das Verschlusselement 2 ist ausgebildet um in einem Herzohreingang angeordnet zu werden und einen Eintritt von Blut in das Herzohr und/oder einen Übertritt von Thromben aus dem Herzohr heraus zu verhindern. Dabei ist das Verschlusselement 2 derart ausgebildet, dass es im implantierten Zustand nicht aus dem Herzohr in den Vorhof des Herzens herausragt.

Wie ebenfalls in der Fig. 1 dargestellt ist das Verschlusselement 2 einstückig mit dem ersten Verankerungselement 3 ausgebildet, welches benachbart zu dem Verschlusselement 2 angeordnet ist. Das erste Verankerungselement 3 ist zur Verankerung an einer Herzohrwandung ausgebildet.

Das Verschlusselement 2 kann ein erstes Membranelement 6 umfassen, wobei das erste Membranelement 6 undurchlässig für Thromben ist, beispielsweise porös. Alternativ oder zusätzlich kann das Verschlusselement 2 als zweites Membranelement 6 umfassen, wobei das zweite Membranelement 6 ausgebildet ist um ein überwuchern des Verschlusselement 2 mit Gewebe zu fördern. Das erste Membranelement 6 und das zweite Membranelement 6 können auch einstückig ausgebildet sein, wie in Fig. 2 dargestellt. Das Membranelement 6 umfasst wenigstens eins der folgenden Materialien: Polyester, Polytetrafluorethlen (PTFE), ePTFE, Teflon, Filz, Goretex, Silikonurethan, Metallfasern und Polypropylen.

Das Membranelement 6 ist derart ausgebildet, dass es im implantierten Zustand der Vorrichtung 1 den Herzohreingang vollständig abdeckt. Dazu bedeckt das Membranelement 6 die dem Vorhof des Herzens zugewandte Oberfläche des Verschlusselements 2 vollständig. Ferner umschließt das Membranelement 6 den Rand des Verschlusselements 2 derart, dass das Membranelement 6 im implantierten Zustand der Vorrichtung 1 vollständig an der Herzohrwandung anliegt. Dazu ist das Membranelement 6 im Bereich des kegelstumpfförmigen Verschlusselements 2 an den konischen Seitenflächen des kegelstumpfförmigen Abschnitts des Verschlusselements 2 angeordnet. Das Membranelement 6 kann sowohl außen an dem Verschlusselement 2 angeordnet werden, wie auch innerhalb des Verschlusselements 2, direkt benachbart zu der Wandung des Verschlusselements 2. In dem Ausführungsbeispiel aus Fig. 2 ist das Membranelement 6 innerhalb des Verschlusselements 2 angeordnet, unmittelbar benachbart zu den drahtartigen Element 9 aus welchem das Verschlusselement 2 gebildet wird. Dadurch dass das Verschlusselement 2 maschenförmig ausgebildet ist, gelangt das Membranelement 6 mit der Wandung des Herzohres in Kontakt, wodurch insbesondere die Überwucherung mit Gewebe gefördert wird.

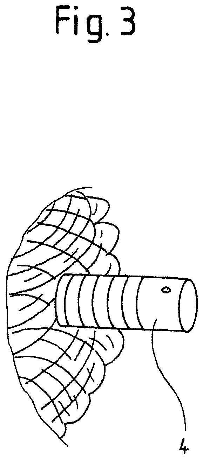

Fig. 3 zeigt eine Detailansicht eines Verbindungselements 4 einer erfindungsgemäßen Vorrichtung 1 zum Verschließen eines Herzohres, insbesondere des linken Herzohres. Das Verbindungselement 4 ist in radialer Richtung flexibel ausgebildet und an einem ersten Ende mit dem ersten Verankerungselement 3 verbunden und an einem zweiten Ende, dem ersten Ende gegenüberliegend, mit dem zweiten Verankerungselement 5 verbunden. Das Verbindungselement 4 ist in radialer Richtung flexibel ausgebildet, damit sich die erfindungsgemäße Vorrichtung 1 an die Anatomie eines Herzohres anpassen kann.

Das Verbindungselement 4 aus Fig. 3 ist aus einem Rohr hergestellt, durch Schneiden von Durchbrechungen in der Wandung des Rohrs, beispielsweise mittels eines Lasers. Durch das Schneiden der Durchbrechungen in dem Rohr ist das Verbindungselement 4 zumindest über einen Teilbereich seiner Länge spiralförmig ausgebildet.

Die Verbindung zwischen den Verbindungselement 4 und dem ersten Verankerungselement 3 beziehungsweise dem zweiten Verankerungselement 5 ist vorzugsweise lösbar ausgebildet. Eine derartige lösbare Verbindung wird beispielsweise durch Durchbrechungen in der Wandung des Verbindungselements 4 ausgebildet, durch welche Riegeldrähte durch das innere des Verbindungselements 4 und in die Wandung des ersten Verankerungselements 3 beziehungsweise des zweiten Verankerungselements 5 geführt werden können. Dazu werden beispielsweise im Bereich des ersten Verankerungselements 3 beziehungsweise des zweiten Verankerungselements 5 innerhalb des Hohlraums innerhalb des Verbindungselements 4 eingeführt, bis sie im Bereich der Durchbrechungen angeordnet sind. Nachfolgend wird durch die Durchbrechungen ein Riegeldraht durchgeführt, welcher gleichzeitig Bereiche der Wandung des ersten Verankerungselements 3 beziehungsweise zweiten Verankerungselements 5 durchtritt. Vor der Implantation der erfindungsgemäßen Vorrichtung 1 werden die Riegeldrähte verschweißt oder verknotet, so dass sich die Verbindung im implantierten Zustand der Vorrichtung 1 nicht mehr lösen kann.

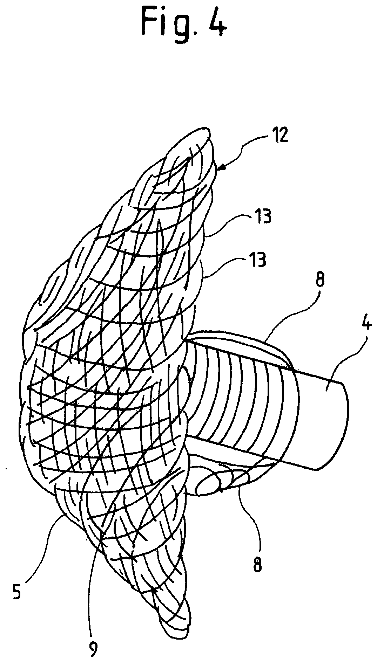

Fig. 4 zeigt eine Detailansicht eines zweiten Verankerungselements 5. Das zweite Verankerungselement 5 wird beispielsweise aus einem einzigen drahtartigen Element 9 gebildet. In dem dargestellten Ausführungsbeispiel aus Fig. 4 ist das zweite Verankerungselement 5 doppellagig ausgebildet, wobei jede Lage aus einem einzigen drahtartigen Element 9 gebildet ist. Die beiden Lagen des zweiten Verankerungselements 5 sind an deren Rändern 12 miteinander verflochten. Der äußere Rand 12 des zweiten Verbindungselements 5 wird durch Schlingen oder Schlaufen 13 gebildet, woraus sich ein wellenförmiger äußerer Rand 12 ergibt. Ein derartiger wellenförmiger äußerer Rand 12 ist besonders zur Verankerung an einer Herzohrwandung ohne eine Perforation der Herzohrwandung geeignet, da ein derartiger wellenförmiger äußerer Rand 12 keine scharfen Kanten aufweist. Das zweite Verankerungselement 5 ist schirmförmig ausgebildet, wobei der Hohlraum des Schirms in Richtung des Herzohreingangs ausgerichtet ist. Bei einer Vorrichtung zum Verschließen eines Herzohrs besteht üblicherweise lediglich die Gefahr, dass die Vorrichtung 1 sich aus dem Herzohr in Richtung des Vorhofs des Herzens bewegt. Eine derartige Bewegung wird durch ein schirmförmiges Verankerungselement 3, 5 wirkungsvoll dadurch vermieden, dass der Hohlraum des schirmförmigen Elements in Richtung des Herzohreingangs ausgerichtet wird. Gemäß Fig. 4 ist die zweite Verbindung 8 zwischen dem zweiten Verankerungselement 5 und dem Verbindungselement 4 ausgebildet durch ein Hindurchführen der drahtartigen Elemente 9 des zweiten Verankerungselements 5 durch Öffnungen in der Umfangswandung des Verbindungselements.

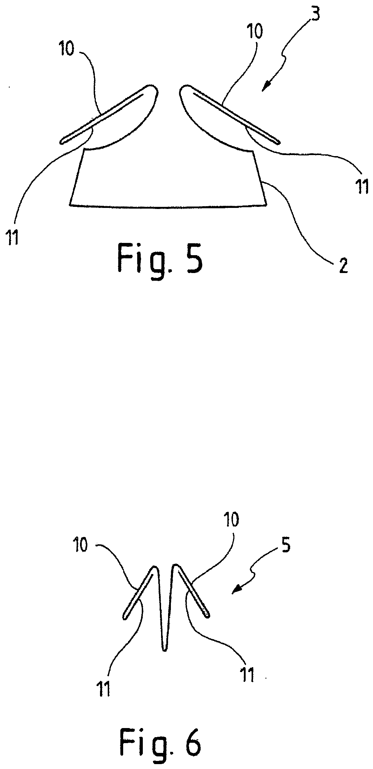

Fig. 5 zeigt eine schematische Ansicht eines weiteren Verschlusselements 2 mit einem ersten Verankerungselement 3. Das in Fig. 5 dargestellte erste Verankerungselement 3 unterscheidet sich von dem ersten Verankerungselement 3 aus den Fig. 1 und 2 dadurch, dass das erste Verankerungselement 3 einen ersten Teilabschnitt 10 und einen zweiten Teilabschnitt 11 aufweist, wobei sich der erste Teilabschnitt 10 in radialer Richtung nach außen erstreckt und der zweite Teilabschnitt 11 in radiale Richtung nach innen zurückgefaltet ist. Wie der Fig. 5 entnommen werden kann sind der erste Teilabschnitt 10 und der zweite Teilabschnitt 11 doppellagig aufeinander gefaltet, wodurch sich die Stabilität des ersten Verankerungselements 3 deutlich erhöht, was zu einer besseren Verankerung des ersten Verankerungselements 3 an der Herzohrwandung führt.

Fig. 6 zeigt eine schematische Ansicht eines weiteren zweiten Verankerungselements 5 einer Vorrichtung zum Verschließen eines Herzohrs, insbesondere des linken Herzohrs. Das zweite Verankerungselement 5 aus Fig. 6 unterscheidet sich von den zweiten Verankerungselementen 5 aus den Fig. 1 und 4 dadurch, dass das zweite Verankerungselement 5 einen ersten Teilabschnitt 10 und einen zweiten Teilabschnitt 11 aufweist, wobei sich der erste Teilabschnitt 10 in radialer Richtung nach außen erstreckt und der zweite Teilabschnitt 11 in radialer Richtung nach innen zurückgefaltet ist, insbesondere derart, dass der erste Teilabschnitt 10 und der zweite Teilabschnitt 11 doppellagig aufeinander gefaltet sind. Das zweite Verankerungselement 5 aus Fig. 6 ist schirmförmig ausgebildet, wobei die Öffnung des Schirms in Richtung des Herzohreingangs ausgerichtet ist.

Die Zurückfaltung des zweiten Teilabschnitts 11 auf den ersten Teilabschnitt 10 aus den Fig. 5 und 6 ist in Richtung des Verschlusselements 2 angeordnet.

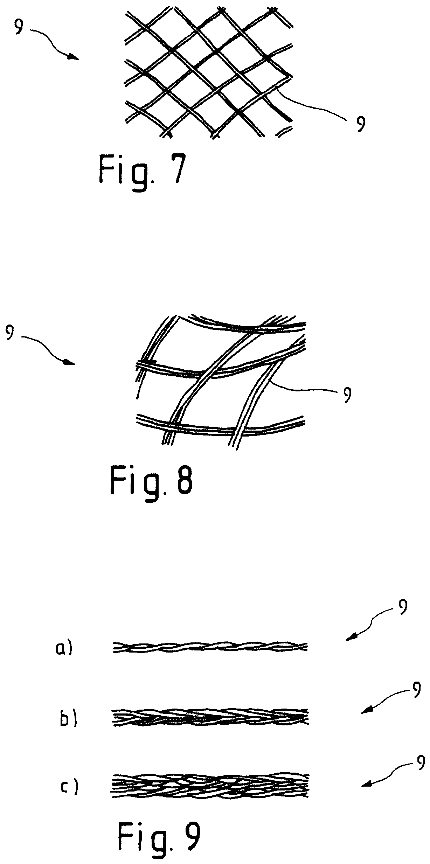

Fig. 7 zeigt eine schematische Ansicht einer Struktur eines Verschlusselements 2 und/oder Verankerungselements 3, 5. Wird das Verschlusselement 2, dass erste Verankerungselement 3 und/oder das zweite Verankerungselement 5 aus einem einzigen drahtartigem Element 9 oder mehreren drahtartigen Elementen durch verschränkendes Wickeln und/oder Verwinden und/oder Verflechten nach Art eines Gewebes und/oder Geleges und/oder Netzes geformt, ergibt sich beispielsweise eine Struktur gemäß Fig. 7. In Fig. 7 ist das Gewebe und/oder Gelege und/oder Netz aus einem einzigen drahtartigen Element 9 geformt.

Fig. 8 zeigt eine schematische Ansicht einer weiteren Struktur eines Verschlusselements 2 und/oder Verankerungselements 3, 5, wobei die Struktur aus einem einzigen drahtartigen Element 9 gebildet wird, wobei das einzige drahtartige Element 9 aus mehreren parallel zueinander verlaufenden Drähten besteht.

Die Fig. 9a bis 9c zeigen eine weitere schematische Ansicht von Ausgestaltungen eines drahtartigen Elements 9, wobei das drahtartige Element 9 aus mehreren miteinander verflochtenen oder verdrillten Einzeldrähten besteht, sogenannten Drahtlitzen oder Litzen.

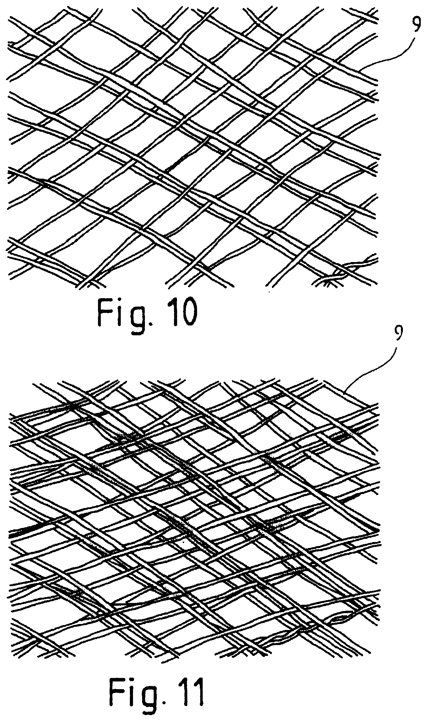

Wie in den Fig. 10 und 11 dargestellt, kann das Verschlusselement 2 und/oder das Verankerungselement 3, 5 doppellagig (Fig. 10) oder mehrlagig (Fig. 11) ausgebildet werden. Dies erhöht die Stabilität der Vorrichtung 1. Ferner können beispielsweise zwischen den einzelnen Lagen ein oder mehrere Membranelemente 6 angeordnet werden.

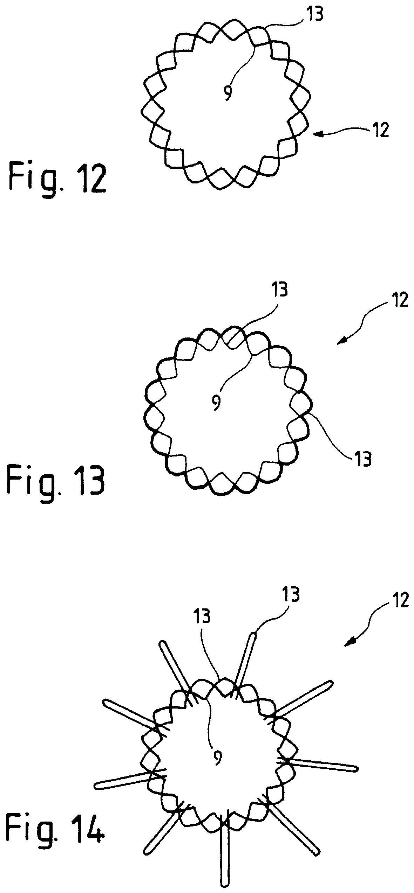

Fig. 12 zeigt eine schematische Ansicht einer ersten Ausgestaltung eines äußeren Randes 12 eines Verankerungselements 3, 5. Der äußere Rand 12 wird aus Schlingen oder Schlaufen 13 gleicher Größe gebildet, woraus sich ein wellenförmiger äußerer Rand 12 ergibt. Ein derartiger wellenförmiger äußerer Rand 12 verbessert die Verankerung des Verankerungselements 3, 5 an der Herzohrwandung.

Fig. 13 zeigt eine schematische Ansicht einer zweiten Ausgestaltung eines äußeren Randes 12 eines Verankerungselements 3, 5. Der äußere Rand 12 gemäß Fig. 13 unterscheidet sich von dem äußeren Rand 12 gemäß Fig. 12 dadurch, dass im Bereich des äußeren Randes 12 eine Materialverstärkung vorgesehen ist. Die Materialverstärkung kann beispielsweise direkt in dem drahtartigen Element 9 vorgesehen sein oder durch eine Überlagerung mehrerer drahtartiger Elemente 9 im Bereich der Schlingen oder Schlaufen 13 erzielt werden.

Fig. 14 zeigt eine schematische Ansicht einer dritten Ausgestaltung eines äußeren Randes 12 eines Verankerungselements 3, 5. Der äußere Rand 12 des Verankerungselements 3, 5 wird durch Schlingen oder Schlaufen 13 unterschiedlicher Größe gebildet. Die Anordnung der unterschiedlichen Größen der Schlingen oder Schlaufen 13 ist dabei gleichmäßig. In Fig. 14 ist eine große Schlinge oder Schlaufe 13 nach zwei kleinen Schlingen oder Schlaufen 13 vorgesehen. Eine derartige Ausgestaltung mit unterschiedlichen Größen der Schlingen oder Schlaufen 13 eignet sich insbesondere in Kombination mit einer Zurückfaltung von einem Teilabschnitt 11 auf einen anderen Teilabschnitt 10, wobei der zurückgefaltete Teilabschnitt 11 durch die großen Schlingen oder Schlaufen 13 gebildet wird. Bei der Zurückfaltung der großen Schlingen oder Schlaufen 13 können diese in die Muskelfasern innerhalb des Herzohrs eingreifen, wodurch die Verankerung der erfindungsgemäßen Vorrichtung 1 in dem Herzohr deutlich verbessert wird.

Auch könnte wie gemäß Fig. 13 der äußere Rand 12 Verstärkungen aufweisen.

### Bezugszeichenliste

- 1: Vorrichtung
- 2: Verschlusselement
- 3: erstes Verankerungselement
- 4: Verbindungselement
- 5: zweites Verankerungselement
- 6: Membranelement
- 7: erste Verbindung
- 8: zweite Verbindung
- 9: drahtartiges Element
- 10: erster Teilabschnitt
- 11: zweiter Teilabschnitt
- 12: äußerer Rand
- 13: Schlingen und/oder Schlaufen

## Patentansprüche

1. Vorrichtung (1) zum Verschließen eines Herzohrs, insbesondere des linken Herzohrs, umfassend:
- ein Verschlusselement (2), ausgebildet um in einem Herzohreingang angeordnet zu werden und einen Eintritt von Blut in das Herzohr und/oder einen Austritt von Thromben aus dem Herzohr heraus zu verhindern,
- ein erstes Verankerungselement (3), welches benachbart zu dem Verschlusselement (2) angeordnet und mit dem Verschlusselement (2) verbunden ist, wobei das erste Verankerungselement (3) zur Verankerung an einer Herzohrwandung ausgebildet ist;
- ein zumindest in radialer Richtung flexibel ausgebildetes Verbindungelement (4), welches mit dem ersten Verankerungselement (3) verbunden ist; und
- ein zweites Verankerungselement (5), welches mit dem Verbindungselement (4) verbunden ist und zur Verankerung an der Herzohrwandung ausgebildet ist,
wobei das erste Verankerungselement (3) scheibenförmig oder schirmförmig ausgebildet ist und sich in radialer Richtung erstreckt,
wobei das Verschlusselement (2) zylindrisch, kegelförmig, konisch zulaufend, kegelstumpfförmig, zylindrisch-kegelförmig, zylindrisch-konisch zulaufend oder zylindrisch-kegelstumpfförmig ausgebildet ist und
**dadurch gekennzeichnet, dass**
sich das Verschlusselement (2) über eine Länge von 0,5 cm bis 2,5 cm in Längsrichtung der Vorrichtung (1) erstreckt, und der Durchmesser des Verschlusselements (2) zwischen 5 % und 20 % größer ist als der Herzohreingang, und dadurch, dass
die dem Vorhof des Herzens zugewandte Oberfläche des Verschlusselements (2) eben ausgebildet ist und im implantierten Zustand nicht aus dem Herzohr in den Vorhof des Herzens herausragt, und dadurch, dass
das Verbindungselement (4) aus einem Rohr hergestellt ist.

2. Vorrichtung (1) nach Anspruch 1, wobei das Verschlusselement (2) ein erstes Membranelement (6) umfasst, wobei das erste Membranelement (6) undurchlässig für Thromben ist.

3. Vorrichtung (1) nach einem der Ansprüche 1 bis 2, wobei das Verschlusselement (2) ein zweites Membranelement (6) umfasst, wobei das zweite Membranelement (6) ausgebildet ist um ein Überwuchern des Verschlusselements (2) mit Gewebe zu fördern.

4. Vorrichtung (1) nach einem der Ansprüche 2 bis 3, wobei das erste Membranelement (6) und/oder das zweite Membranelement (6) im implantierten Zustand der Vorrichtung (1) den Herzohreingang vollständig abdeckt.

5. Vorrichtung (1) nach Anspruch 4, wobei das erste Membranelement (6) und/oder das zweite Membranelement (6) den Rand des Verschlusselements (2) derart umschließt, dass das erste Membranelement (6) und/oder das zweite Membranelement (6) im implantierten Zustand der Vorrichtung (1) vollständig an der Herzohrwandung anliegt.

6. Vorrichtung (1) nach einem der Ansprüche 1 bis 5, wobei das Verschlusselement (2) und das erste Verankerungselement (3) fest miteinander verbunden sind oder einstückig ausgebildet sind.

7. Vorrichtung (1) nach einem der Ansprüche 1 bis 6, wobei das Verbindungselement (4) durch Schneiden von Durchbrechungen in dem Rohr, beispielsweise mittels eines Lasers, hergestellt ist.

8. Vorrichtung (1) nach einem der Ansprüche 1 bis 7, wobei das Verschlusselement (2), das erste Verankerungselement (3) und/oder das zweite Verankerungselement (5) aus einem einzigen drahtartigen Element (9) oder mehreren drahtartigen Elementen durch verschränkendes Wickeln und/oder Verwinden und/oder Verflechten nach Art eines Gewebes und/oder Geleges und/oder Netzes geformt ist.

9. Vorrichtung (1) nach einem der Ansprüche 1 bis 8, wobei das zweite Verankerungselement (5) scheibenförmig oder schirmförmig ausgebildet ist und sich vorzugsweise in radialer Richtung erstreckt.

10. Vorrichtung (1) nach einem der Ansprüche 1 bis 9, wobei das erste Verankerungselement (3) und/oder das zweite Verankerungselement (5) bauchig und/oder gekrümmt gebildet ist.

11. Vorrichtung (1) nach einem der Ansprüche 1 bis 10, wobei der Durchmesser des ersten Verankerungselements (3) gleich dem Durchmesser des zweiten Verankerungselements (5) ist oder der Durchmesser des ersten Verankerungselements (3) größer ist als der Durchmesser des zweiten Verankerungselements (5).

12. Vorrichtung (1) nach einem der Ansprüche 1 bis 11, wobei die Vorrichtung (1) replatzierbar und/oder explantierbar ausgebildet ist, beispielsweise durch ein Kopplungselement für ein Implantationsinstrument.

13. Vorrichtung (1) nach einem der Ansprüche 1 bis 12, wobei die Vorrichtung (1) in einem ersten Betriebszustand (Primärform) ein großes Verhältnis von Länge zu Querausdehnung entlang der Längsachse der Vorrichtung (1) und in einem zweiten Betriebszustand (Sekundärform) ein kleineres Verhältnis von Länge zu Querausdehnung entlang der Längsachse der Vorrichtung (1) aufweist und die Vorrichtung (1) durch Aufbringen einer Kraft gegen elastische Materialkräfte aus der Sekundärform in die Primärform reversibel überführbar ist.

## Claims

1. Device (1) for closing an atrial appendage, particularly the left atrial appendage, comprising:
- a closing element (2), configured to be arranged in an atrial appendage entrance and to prevent an entrance of blood into the atrial appendage and/or a leakage of thrombus out of the atrial appendage,
- a first anchoring element (3), adjacent to the closing element (2) and connected to the closing element (2), wherein the first anchoring element (3) is configured for anchoring on the atrial appendage wall,
- a connecting element (4), which is at least in a radial direction flexible and connected to the first anchoring element (3), and
- a second anchoring element (5), which is connected to the connecting element (4) and configured for anchoring on the atrial appendage wall,
wherein the first anchoring element (3) is disc-shaped or umbrella-shaped and extends in a radial direction,
wherein the closing element (2) is cylindrical, conical, tapered, frustoconical, cylindrically conical, cylindrically conically tapered or cylindrically frustoconical, and
**characterized in that**
the closing element (2) extends over a length of 0,5 cm to 2,5 cm in the longitudinal direction of the device (1), and the diameter of the closing element (2) is between 5 % and 20 % larger than the atrial appendage entrance, and **in that**
the surface of the closing element (2) facing the atrium of the heart is smooth and in the implanted state does not protrude from the atrial appendage into the atrium of the heart, and **in that**
the connecting element (4) is made of a tube.

2. Device (1) according to claim 1, wherein the closing element (2) comprises a first membrane element (6), wherein the first membrane element (6) is impermeable to thrombi.

3. Device (1) according to claim 1 or claim 2, wherein the closing element (2) comprises a second membrane element (6), wherein the second membrane element (6) is configured to support an overgrow of the closing element (2) with endothelial tissue.

4. Device (1) according to one of claims 2 to 3, wherein the first membrane element (6) and/or the second membrane element (6) completely cover the atrial appendage entrance in the implanted state of the device (1).

5. Device (1) according to claim 4, wherein the first membrane element (6) and/or the second membrane element (6) enclose the rim of the closing element (2) in such a way, that the first membrane element (6) and/or the second membrane element (6) completely contacts the atrial appendage wall in the implanted state of the device (1).

6. Device (1) according to one of claims 1 to 5, wherein the closing element (2) and the first anchoring element (3) are fixed securely together or are formed integrally.

7. Device according to one of claims 1 to 6, wherein the connecting element (4) is built by cutting apertures into the tube, for example using a laser.

8. Device (1) according to one of claims 1 to 7, wherein the closing element (2), the first anchoring element (3) and/or the second anchoring element (5) is formed from a single wire-like element (9) or multiple wire-like elements by interlocking winding and/or twisting and/or interlacing in the manner of a fabric and/or scrim and/or net.

9. Device (1) according to one of claims 1 to 8, wherein the second anchoring element (5) is disc-shaped or umbrella-shaped and extends preferably in a radial direction.

10. Device (1) according to one of claims 1 to 9, wherein the first anchoring element (3) and/or the second anchoring element (5) is bulbous and/or curved.

11. Device (1) according to one of claims 1 to 10, wherein the diameter of the first anchoring element (3) is equal to the diameter of the second anchoring element (5) or the diameter of the first anchoring element (3) is larger than the diameter of the second anchoring element (5).

12. Device (1) according to one of claims 1 to 11, wherein the device (1) is relocatable and/or explantable, for example by means of a coupling element for an implantation instrument.

13. Device (1) according to one of claims 1 to 12, wherein the device (1) has, in a first operating state (primary form), a large ratio of length to lateral extension along a longitudinal axis of the device (1), and, in a second operating state (secondary form), a smaller ratio of length to lateral extension along the longitudinal axis of the device (1), and the device (1) can be reversibly deformed against elastic material forces from the secondary form to the primary form.

## Revendications

1. Dispositif (1) servant à fermer un appendice auriculaire, en particulier l'appendice auriculaire gauche, comprenant :
- un élément de fermeture (2), configuré pour être agencé dans une entrée de l'appendice auriculaire et pour empêcher une entrée de sang dans l'appendice auriculaire et/ou une fuite de thrombus hors de l'appendice auriculaire,
- un premier élément d'ancrage (3), adjacent à l'élément de fermeture (2) et relié à l'élément de fermeture (2), le premier élément d'ancrage (3) étant configuré pour s'ancrer sur la paroi de l'appendice auriculaire,
- un élément de liaison (4), qui est au moins dans une direction radiale flexible et relié au premier élément d'ancrage (3), et
- un deuxième élément d'ancrage (5), qui est relié à l'élément de liaison (4) et configuré pour s'ancrer sur la paroi de l'appendice auriculaire,
le premier élément d'ancrage (3) étant en forme de disque ou de parapluie et s'étendant dans une direction radiale,
l'élément de fermeture (2) étant cylindrique, conique, effilé, tronconique, cylindriquement conique, cylindriquement effilé de manière conique, ou cylindriquement tronconique, et
**caractérisé en ce que**
l'élément de fermeture (2) s'étend sur une longueur de 0,5 cm à 2,5 cm dans la direction longitudinale du dispositif (1), et le diamètre de l'élément de fermeture (2) se situe entre 5 % et 20 % supérieur à l'entrée de l'appendice auriculaire, et **en ce que**
la surface de l'élément de fermeture (2) faisant face à l'oreillette du coeur est lisse et, dans l'état implanté, ne dépasse pas de l'appendice auriculaire dans l'oreillette du coeur, et **en ce que**
l'élément de liaison (4) est composé d'un tube.

2. Dispositif (1) selon la revendication 1, l'élément de fermeture (2) comprenant un premier élément de membrane (6), le premier élément de membrane (6) étant imperméable aux thrombus.

3. Dispositif (1) selon la revendication 1 ou la revendication 2, l'élément de fermeture (2) comprenant un deuxième élément de membrane (6), le deuxième élément de membrane (6) étant configuré pour supporter une surcroissance de l'élément de fermeture (2) avec un tissu endothélial.

4. Dispositif (1) selon l'une des revendications 2 à 3, le premier élément de membrane (6) et/ou le deuxième élément de membrane (6) recouvrant complètement l'entrée de l'appendice auriculaire dans l'état implanté du dispositif (1).

5. Dispositif (1) selon la revendication 4, le premier élément de membrane (6) et/ou le deuxième élément de membrane (6) entourant le bord de l'élément de fermeture (2) de telle façon que le premier élément de membrane (6) et/ou le deuxième élément de membrane (6) viennent complètement en contact avec la paroi de l'appendice auriculaire dans l'état implanté du dispositif (1).

6. Dispositif (1) selon l'une des revendications 1 à 5, l'élément de fermeture (2) et le premier élément d'ancrage (3) étant fixés solidement ensemble ou étant formés d'un seul tenant.

7. Dispositif selon l'une des revendications 1 à 6, l'élément de liaison (4) étant élaboré par découpe d'ouvertures dans le tube, par exemple en utilisant un laser.

8. Dispositif (1) selon l'une des revendications 1 à 7, l'élément de fermeture (2), le premier élément d'ancrage (3) et/ou le deuxième élément d'ancrage (5) étant formés à partir d'un élément unique de type fil (9) ou de multiples éléments de type fil en verrouillant l'enroulement et/ou la torsion et/ou l'entrelacement à la manière d'un tissu et/ou d'un canevas et/ou d'un filet.

9. Dispositif (1) selon l'une des revendications 1 à 8, le deuxième élément d'ancrage (5) étant en forme de disque ou de parapluie et s'étendant de préférence dans une direction radiale.

10. Dispositif (1) selon l'une des revendications 1 à 9, le premier élément d'ancrage (3) et/ou le deuxième élément d'ancrage (5) étant bulbeux et/ou incurvés.

11. Dispositif (1) selon l'une des revendications 1 à 10, le diamètre du premier élément d'ancrage (3) étant égal au diamètre du deuxième élément d'ancrage (5) ou le diamètre du premier élément d'ancrage (3) étant supérieur au diamètre du deuxième élément d'ancrage (5).

12. Dispositif (1) selon l'une des revendications 1 à 11, le dispositif (1) étant adapté pour être repositionné et/ou explanté, par exemple au moyen d'un élément de couplage destiné à un instrument d'implantation.

13. Dispositif (1) selon l'une des revendications 1 à 12, le dispositif (1) ayant, dans un premier état de fonctionnement (forme primaire), un grand rapport de longueur à extension latérale le long d'un axe longitudinal du dispositif (1), et, dans un deuxième état de fonctionnement (forme secondaire), un rapport inférieur de longueur à extension latérale le long de l'axe longitudinal du dispositif (1), et le dispositif (1) pouvant être déformé de manière réversible par rapport à des forces de matériau élastique de la forme secondaire à la forme primaire.
